# EUROPEAN PATENT APPLICATION

(11) **EP 1 602 730 A2**
(43) Date of publication of application: **07.12.2005**
(21) Application number: 04076644.6
(22) Date of filing: 04.06.2004
(51) Int. Cl.: C12P 13/00, C12P 7/42, C12N 1/14, C12N 1/20, C12P 13/04

(54) **Biosynthetic production of 4-amino-4-deoxychorismate (ADC) and [3R,4R]-4-amino-3-hydroxycyclohexa-1,5-diene-1-carboxylic acid (3,4-CHA)**

(71) Applicant: DSM IP Assets B.V., 6411 TE Heerlen (NL); DSM Biotech GmbH, 52428 Jülich (DE); FORSCHUNGSZENTRUM JÜLICH GMBH, 52425 Jülich (DE)
(72) Inventor: Wubbolts, Marcel Gerhardus, 6132 CX Sittard (NL); Bongaerts, Johannes Josef, 50259 Pulheim (DE); Bovenberg, Roelef Ary Lans, 3062 DA Rotterdam (NL); Kozak, Stefan, 70178 Stuttgart (DE); Sprenger, Georg, 71522 Backnan-Maubach (DE); Müller, Michael, 52425 Jülich (DE)
(74) Representative: Verhaegen, Ilse Maria M.

(57) **Abstract**

The invention relates to a process for the biosynthetic production of 4-amino-4-deoxychorismate (ADC) performed fermentatively *in vivo* with a 4-amino-4-deoxychorismate synthase, preferably a PabAB bipartite protein (which may be a fusion protein), at an increased level of activity, thereby obtaining a broth comprising ADC and 4-amino-4-deoxyprephenate (ADP), that are recovered. The invention also relates to a further process of converting the ADP into p-aminophenylalanine. The invention, moreover relates to biosynthetic production of [3*R*,4*R*]-4-amino-3-hydroxycyclohexa-1,5-diene-1-carboxylic acid (3,4-CHA), by concerted action of such 4-amino-4-deoxychorismate synthase and of an enzyme capable of converting isochorismate into [5*S*,6*S*]-5,6-dihydroxycyclohexa-1,3-diene-1-carboxylic acid (2,3-CHD), preferably a phenazine biosynthesis protein PhzD, including recovery of 3,4-CHA. The invention also relates to expression vectors and host cells for use in any of such processes. And the invention finally relates to synthesis of oseltamivir phosphate from 3,4-CHA.

## Description

The present invention relates, in a first embodiment thereof, to a process for the biosynthetic production of 4-amino-4-deoxychorismate (ADC) catalyzed at least by an enzyme belonging to the class of aminodeoxychorismate synthases. In said process a mixture of 4-amino-4-deoxychorismate (ADC) and 4-amino-4-deoxyprephenate (ADP) is formed in a fermentation broth. In a subsequent step of said embodiment, the invention also relates to the synthesis of p-aminophenylalanine.

The term biosynthetic production as used herein is used in its broadest possible meaning, unless it is clear from the context that a narrower meaning is intended. It includes processes that are carried out fermentatively *in vivo,* as well as processes that are carried out *in vitro*. Generally, *in vivo* processes are processes carried out when using living cells (the term "living cells" thereby also including so-called resting cells); *in vitro* processes, on the other hand, usually are being carried out using cell lysates or (partly) purified enzymes. The biochemical production as meant herein, however, also may be carried out using permeabilized cells; the differentiation between *in vivo* and *in vitro,* however, does not make much sense for processes being carried out with permeabilized cells. It will be evident, that the process of the invention also can be carried out using immobilized host cells, immobilized enzymes, etc. In case claims are restricted to "*in vivo"* processes, this will be indicated specifically. Although, strictly speaking from a technical point of view, the term "fermentatively" often would mean that a process is carried out without oxygen or under limited oxygen supply, the term is used in the context of the present patent application in the broadest possible sense and includes aerobic processes as well. It intends to indicate that all general techniques available for the fermentation technologist can be used in the methods according to the present invention.

The present invention also, in a second embodiment, relates to a process for the biosynthetic production of [3*R*,4*R*]-4-amino-3-hydroxycyclohexa-1,5-diene-1-carboxylic acid (3,4-CHA). This compound [3*R*,4*R*]-4-amino-3-hydroxycyclohexa-1,5-diene-1-carboxylic acid (3,4-CHA) can also be referred to as *trans*-4-amino-3-hydroxycyclohexa-1,5-diene-1-carboxylic acid.

Further, the present invention also relates to host cells, expression vectors, plasmids and so on as can be used in the processes of any of the embodiments of the present invention.

Finally, the present invention also relates to the further conversion of [3*R*,4*R*]-4-amino-3-hydroxycyclohexa-1,5-diene-1-carboxylic acid (3,4-CHA), as can be produced according to the present invention. In particular, it relates to the further conversion in a novel synthesis route for the production of a product called oseltamivir phosphate (IUPAC name: ethyl (3*R*,4*R*,5*S*)-4-*N*-acetyl-amino-3-(1-ethylpropoxy)-5-*N*-amino-1-cyclohexene-1-carboxylate phosphate [1:1]), which is the active ingredient for an anti-influenza pharmaceutical product known under the trade name Tamiflu® of Hoffmann-La Roche, Switzerland.

Biosynthetic production of 4-amino-4-deoxychorismate (ADC) is known from K. S. Anderson *et al*., JACS 113 (1991), pages 3198-3200. Parsons *et al*., in Biochem. 42, p.5684-5693 (2003), describe at page 5690 that ADC is only barely hydrolyzed under the influence of the phenazine biosynthesis PhzD protein for which it apparently is a poor substrate. Biosynthesis of [3*R*,4*R*]-4-amino-3-hydroxycyclohexa-1,5-diene-1-carboxylic acid (3,4-CHA) so far has not been described at all. For the synthesis of oseltamivir phosphate so far only quite laborious methods are available comprising more than 10 reaction steps. Reference, for instance can be made to a 12-step method starting from quinic acid, as described by Rohloff *et* al., J. Org. Chem., 63, p.4545-4550 (1999); or to an 11-step method starting from shikimic acid as described by M. Federspiel *et al*., Organic Process Research & Development, 3, p. 266-274 (1999).

Biosynthetic production of 4-amino-4-deoxyprephenate (ADP) has never been described as a subject of detailed studies, presumably because the product ADP, like it is known for ADC, is believed to be unstable.

Disadvantages of the known methods are:
a) for the biosynthetic production of aminodeoxychorismate (ADC) so far only synthesis at mg-scale, and at very low concentrations of a few mg/l, has been described; thus, recovery of ADC from such reaction mixtures is very difficult;
b) for the biosynthetic production of [3*R*,4*R*]-4-amino-3-hydroxycyclohexa-1,5-diene-1-carboxylic acid (3,4-CHA) no method is yet available at all;
c) for the state-of-the-art synthesis of oseltamivir phosphate so far a more than 10 step reaction sequence is needed.

Accordingly there is need for an improved process for the biosynthesis of aminodeoxychorismate (ADC), for a completely novel biosynthetic process for the synthesis of [3*R*,4*R*]-4-amino-3-hydroxycyclohexa-1,5-diene-1-carboxylic acid (3,4-CHA), and for an improved process for the synthesis of oseltamivir phosphate.

The above problems all have been solved by the present invention, having the basic features as are being claimed in claim 1 hereof.

The present inventors, surprisingly, now have found that the biosynthetic production of 4-amino-4-deoxychorismate (ADC), catalyzed at least by an enzyme belonging to the class of aminodeoxychorismate synthases, is performed fermentatively *in vivo* with a 4-amino-4-deoxychorismate synthase at an increased level of activity, while obtaining a fermentation broth comprising 4-amino-4-deoxychorismate (ADC) and 4-amino-4-deoxyprephenate (ADP), and that these compounds, either together or individually, are recovered from the fermentation broth.

Because ADC synthesis in nature is the first step in folate synthesis from chorismate, aminodeoxychorismate synthase enzymes are abundantly available in nature. They are assumed to be present in all folate prototrophic organisms, e.g. in bacteria, yeasts, plants and lower eukaryotes.

As used herein, the term "at an increased level of activity" means that the level of activity (of any particular enzyme as indicated) is higher than the level of (native) activity of said enzyme in its native surroundings (i.e. natural source cells) under standard conditions. Increasing the activity level can be achieved by all kinds of methods known to the skilled man, e.g. by overexpression of the gene coding for such enzyme, multi-copying of such genes, or providing the genes with improved translational and/or transcriptional efficiency, for instance by means of a stronger promoter, an inducible promoter, etc.

ADC synthases, and in any case those from *Escherichia coli*, are known to exist in the form of single units, but sometimes occur as bipartite enzymes. The single units, for instance, may consist of a so-called PabA protein (a protein known to generate ammonia from glutamine) or of a so-called PabB protein (a protein known to insert ammonia directly into chorismate). As meant herein, the term bipartite enzyme represents any active (i.e. having the functionality of an ADC synthase) protein that combines the functions of PabA and PabB. In principle, the term "bipartite protein" as used herein interchangeably can be used with any of the terms "fusion protein" or "covalently linked protein complex".

G. Bassett *et al*. describe in PNAS, 101, pages 1496-1501 (2004) that bipartite proteins containing fused PabA and PabB domains are involved in the synthesis of ADC in plants. It is to be noticed, that - as is also being done in the article of Bassett *et al.,* these ADC synthases in literature occasionally (and, as the present inventors believe, wrongly) are also referred to as PABA synthases (p-aminobenzoate synthases). The present inventors have shown in their studies that enzymes which incorrectly have been named PABA synthases indeed all lead to the formation of ADC (in admixture with ADP), and that formation of p-aminobenzoate (if observed at all; at most as a minor by-product) hardly occurs in the process embodiments of the present invention. As Bassett *et al.* have confirmed that conversion into p-aminobenzoate requires the additional presence of the protein PabC. Accordingly it is most surprising in the methods of the present invention, that, although the *pabC* may be present, no p-aminobenzoate is formed.

In a preferred embodiment of this first embodiment of the invention the biosynthetic production of 4-amino-4-deoxychorismate (ADC) is performed whereby the 4-amino-4-deoxychorismate synthase is a PabAB bipartite protein.

As meant herein, the term 4-amino-4-deoxychorismate synthase PabAB bipartite protein represents any active protein (i.e. any protein having the functionality of an ADC synthase) that combines the functions of PabA (a protein known to generate ammonia from glutamine) and PabB (a protein known to insert ammonia directly into chorismate). In principle, the term "bipartite protein" as used herein interchangeably can be used with any of the terms "fusion protein" or "covalently linked protein complex".

The process according to the first embodiment of the invention is being carried out *in vivo.* In the processes of the prior art only *in vitro* synthesis is described.

In particular, and preferably, the bipartite protein originates from a species from the group of *Actinomycetes,* or from plants containing such bipartite enzyme, or is constructed by fusion of genes respectively encoding for PabA and PabB. Methods for fusion of genes are well-known to the skilled man, and may, for instance, consist of PCR methods, cloning, etc.

Accordingly, the bipartite protein may be a naturally occurring (bipartite) protein from plants, or an artificially constructed one. The inventors have observed that there is rather high homology between PabA proteins and certain glutamine aminotransferases that are also referred to as TrpD proteins, and that there is also rather high homology between PabB proteins and certain anthranilate synthases that are also referred to as TrpE proteins. Accordingly - instead of, or together with - the PabA resp. PabB parts of the bipartite protein, also TrpD (instead of PabA) and TrpE (instead of PabB) can be applied in the process of this invention.

This means that any of the following combinations

PabA/PabB; TrpD/PabB; PabA/TrpE; and TrpD/TrpE all lead to favorable results according to (this first embodiment of) the present invention. Therefore, all such combinations of enzyme activities are being encompassed in the term 4-amino-4-deoxychorismate synthase PabAB bipartite protein.

Because TrpD and TrpE in nature are involved in the first steps of tryptophan synthesis these enzymes are abundantly available in nature. They are assumed to be present in all L-tryptophane prototrophic organisms, e.g. in bacteria, yeasts, plants and lower eukaryotes.

Accordingly, genes coding for each of the parts of the bipartite proteins PabA/PabB; TrpD/PabB; PabA/TrpE; and TrpD/TrpE are well-known and available to the skilled man.

Preferably, the PabAB bipartite protein originates, as such or as a fusion protein, from a species from the group of genera consisting of *Escherichia, Corynebacterium, Saccharomyces* or *Streptomyces.* In the process according to the invention for the biosynthesis of ADC, the PabAB bipartite protein more preferably originates, as such or as a fusion protein, from one of the species from the group of species consisting of *Escherichia coli, Corynebacterium glutamicum, Corynebacterium diphtheriae gravis* NCTC13129, *Corynebacterium efficiens, Saccharomyces cerevisiae, Streptomyces griseus, Streptomyces venezuelae, Streptomyces sp.* FR-008, *Streptomyces pristinaespiralis, Streptomyces thioluteus,* and *Streptomyces avermitilis*. It is most preferred, that the PabAB bipartite protein originates from *Corynebacterium glutamicum* ATCC 13032.

As mentioned above, the proteins are to be present "at an increased level of activity". This means that the level of activity (of any particular enzyme as indicated) is higher than the level of (native) activity of said enzyme in its native surroundings (i.e. natural source cells) under standard conditions.

Best results are achieved when expression of the PabAB bipartite protein is manipulated in such way that timing of the start of actual production is started after the host microorganism in which the protein is expressed has reached an O.D. (optical density) at 620 nm in the range of from about 0.5 to 100, preferably of at most 50. This expression can be achieved with or without induction. In case the expression is achieved with induction, it is preferably done with help of a strong promoter, for instance a p*tac* promoter, and is induced by isopropyl-β-D-thiogalactopyranoside (IPTG).

In this first embodiment of the present invention, the 4-amino-4-deoxy-chorismate (ADC) and 4-amino-4-deoxyprephenate (ADP) formed are preferably recovered from the said obtained fermentation broth, together or individually, by a separation process selected from the group consisting of reactive extraction and chromatography, optionally followed by crystallization.

In a subsequent step of this first embodiment of the invention, the synthesis of p-aminophenylalanine is achieved. This is preferably being done by converting the 4-amino-4-deoxyprephenate (ADP) in the mixture of 4-amino-4-deoxychorismate (ADC) and 4-amino-4-deoxyprephenate (ADP) in a further step into p-aminophenylalanine by means of concerted action, and at an increased level of activity, of a 4-amino-4-deoxyprephenate dehydrogenase and an aminotransferase, which may be an L- or D-specific aminotransferase from any suitable source. Of course, the p-aminophenylalanine then may be converted in subsequent steps into derivatives thereof.

The 4-amino-4-deoxyprephenate dehydrogenase is preferably a PapC protein. The gene *papC* is, for instance, known from the pristinamycin biosynthesis in *Streptomyces pristinaespiralis* (see Blanc *et al.,* Molec. Microbiol., 23, p.191-202 (1997). The *papC* gene has a close resemblance with the *cmlC* gene (also encoding for 4-amino-4-deoxyprephenate dehydrogenase) as is involved in chloramphenicol synthesis in *Streptomyces venezuelae* (see He *et al.,* Microbiol., 147, p.2817-2829 (2001). For the purposes of the present invention, proteins encoded by *cmlC* are also considered to be PapC proteins.

The conversion of ADP into p-aminophenylalanine is described in a paper of R. A. Mehl *et al.,* JACS 125, p.935-939 (2003), wherein (as shown in Figure 1 at page 936) subsequent action of PapA, PapB and PapC followed by interaction with an *E*. *coli* aminotransferase leads to the formation of p-aminophenylalanine. It is to be noticed, that these authors indeed cloned the *cml* genes from *S*. *Venezuelae* for the biosynthesis of p-aminophenylalanine, but they labeled these genes with *pap* as would have been according to the labeling of the *S*. *Pristinaespiralis* genes. According to the above described embodiment of the present invention the number of enzymes involved, and thus the number of steps, in the conversion into p-aminophenylalanine is one lower than in the route described by Mehl *et al.*

The present invention, in a second embodiment thereof, relates to a process for the biosynthetic production of [3*R*,4*R*]-4-amino-3-hydroxycyclohexa-1,5-diene-1-carboxylic acid (3,4-CHA), wherein the biosynthetic production is performed by concerted action, and at an increased level of activity, of a 4-amino-4-deoxy-chorismate synthase and of an enzyme capable of converting isochorismate into [5*S*,6*S*]-5,6-dihydroxycyclohexa-1,3-diene-1-carboxylic acid (2,3-CHD), and wherein the [3*R*,4*R*]-4-amino-3-hydroxycyclohexa-1,5-diene-1-carboxylic acid (3,4-CHA) is recovered from the fermentation broth so obtained. 2,3-CHD also can be referred to as [2*S*,3*S*]-2,3-dihydroxy-2,3-dihydrobenzoic acid.

In this embodiment of the invention, the biosynthetic production may be carried out either fermentatively *in vivo,* or may be carried out enzymatically *in vitro,* for instance by using enzyme preparations comprising the aforementioned enzyme activities. Such enzyme preparations, for instance, may be present in the form of enzymes on carrier, or in resting cells, or as cell lysates, or as (partly) purified enzymes, or in any other form known to the skilled man.

As meant herein, the phrase "by concerted action, and at an increased level of activity" indicates that both enzymes mentioned act together, and each of them is being used at a level of activity (of such enzyme) that is higher than the level of (native) activity of said enzyme in its native surroundings (i.e. natural source cells) under standard conditions. As indicated before, increasing the activity level of an enzyme can be achieved by all kinds of methods known to the skilled man, e.g. by overexpression of the gene coding for such enzyme, multi-copying of such genes, or providing the genes with improved translational and/or transcriptional efficiency, for instance by means of a stronger promoter, an inducible promoter, etc.

It is noticed that, in principle, and from a chemical point of view, ADC might be considered to be a mono-substituted (i.e. protected) form of [3*R*,4*R*]-4-amino-3-hydroxycyclohexa-1,5-diene-1-carboxylic acid (3,4-CHA). In fact, conversely, ADC also might be called a derivative of 3,4-CHA. ADC and 3,4-CHA both are important intermediates for the synthesis of interesting further compounds.

Preferably, the biosynthetic production of [3*R*,4*R*]-4-amino-3-hydroxy-cyclohexa-1,5-diene-1-carboxylic acid (3,4-CHA) is performed fermentatively *in vivo*.

Suitable enzymes capable of converting isochorismate into [5*S*,6*S*]-5,6-dihydroxycyclohexa-1,3-diene-1-carboxylic acid (2,3-CHD) can be obtained from the enzyme class of isochorismatases [EC 3.3.2.1]. Of course, also mutants and muteins of this class of proteins that have been engineered so as to be optimized for the desired conversion of ADC into 3,4-CHA, even if they are no longer capable of catalyzing the conversion of isochorismate into 2,3-CHD, are - within the context of the present application - considered to fall within the said class of enzymes.

In this second embodiment of the present invention, the 4-amino-4-deoxy-chorismate synthase most preferably is a PabAB bipartite protein. For the PabAB bipartite protein all remarks are applicable as have been made in the foregoing part of this patent application with respect to the first embodiment of the invention.

Preferably, the enzyme capable of converting isochorismate into [5*S*,6*S*]-5,6-dihydroxycyclohexa-1,3-diene-1-carboxylic acid (2,3-CHD) is an isochorismatase enzyme.

In particular, in this embodiment, the PabAB bipartite protein is a protein as described above for the first embodiment of the invention, and the enzyme capable of converting isochorismate into [5*S*,6*S*]-5,6-dihydroxycyclohexa-1,3-diene-1-carboxylic acid (2,3-CHD) is also capable of converting 4-amino-4-deoxyisochorismate into [5*S*,6*S*]-6-amino-5-hydroxycyclohexa-1,3-diene-1-carboxylic acid (2,3-CHA).

[5*S*,6*S*]-6-amino-5-hydroxycyclohexa-1,3-diene-1-carboxylic acid (2,3-CHA) can also be referred to as [2*S*,3*S*]-2-amino-3-hydroxy-2,3-dihydrobenzoic acid, or as *trans*-2,3-dihydro-3-hydroxyanthranilic acid.

It is most preferred, that the enzyme capable of converting isochorismate into [5*S*,6*S*]-5,6-dihydroxycyclohexa-1,3-diene-1-carboxylic acid (2,3-CHD) is a phenazine biosynthesis PhzD protein. [5*S*,6*S*]-5,6-dihydroxycyclo-hexa-1,3-diene-1-carboxylic acid (2,3-CHD) can also be referred to as [2*S*,3*S*]-2,3-dihydroxy-2,3-dihydrobenzoic acid.

Genes coding for PhzD are, for instance, known from an article of McDonald *et al.,* JACS, 123, p.9459-9460 (2001). The phenazine genes have been identified and sequences by D. Mavrodi *et al.,* as described in J. Bacteriol. 180, p.2541-2548 (1998).

It is, moreover, mentioned in an article of J.B Laursen *et al.* in Chem. Rev. 104, p. 1663-1685 (2004), entitled "Phenazine natural products: biosynthesis, synthetic analogues and biological activity", that phenazine natural products are isolated primarily from *Pseudomonas* and *Streptomyces*, and a few other genera from soil or marine habitats. Examples of such other species are: *Methanosarcina mazei* Gö1, *Pelagiobacter variabilis, Vibrio* strains, *Erwinia herbicola* (this is a strain also being named as *Pantoea agglomerans*), *Burkholderia phenazinium, Waksmania aerata,* and *Sorangium* species. Accordingly, proteins homologous to PhzD preferably are being obtained from any of such strains.

Thus, preferably, the phenazine biosynthesis PhzD protein originates from a species from the group of genera consisting of *Pseudomonas, Pantoea, Streptomyces,* and *Erwinia.* In particular, the phenazine biosynthesis PhzD protein originates from a species selected from the group consisting of *Pseudomonas aeruginosa, Pseudomonas aureofaciens, Pseudomonas fluorescens, Pseudomonas chlororaphis,* and *Pantoea agglomerans* species. Most preferably, the phenazine biosynthesis PhzD protein originates from *Pseudomonas aeruginosa* ATCC 17933.

In this second embodiment of the present invention, the PabAB bipartite protein originates, as such or as a fusion protein, from a species from the group of genera consisting of *Escherichia, Corynebacterium, Saccharomyces* or *Streptomyces,* most preferably selected from the group of species consisting of *Escherichia coli, Corynebacterium glutamicum, Corynebacterium diphtheriae gravis* NCTC13129, *Corynebacterium efficiens, Saccharomyces cerevisiae, Streptomyces griseus, Streptomyces venezuelae, Streptomyces sp*.FR-008, *Streptomyces pristinaespiralis, Streptomyces thioluteus,* and *Streptomyces avermitilis*.

Most preferably, the bipartite protein originates from *Corynebacterium glutamicum* ATCC 13032. It is advantageous if the phenazine biosynthesis PhzD protein is tagged at its C- or N-terminus. If so, then the phenazine biosynthesis PhzD protein is preferably tagged with a short tag sequence with from 5 to 15 units, selected from the group consisting of His, Myc and Strep tags. Within this selection of tag groups, the phenazine biosynthesis PhzD protein is preferably His-tagged (with from 5 to 15 His-units) at its N-terminal end.

In addition, it is extremely advantageous if, in this embodiment of the process according to the invention, the expression of the phenazine biosynthesis PhzD protein is controlled by a T7 polymerase promoter upstream of the His-*phzD* gene. This is most surprising, since such effect of the T7 promoter is not to be expected, especially in host strains like *Escherichia coli*, where T7 polymerase is absent. Most preferably, the phenazine biosynthesis PhzD protein is His₁₀-tagged.

[3*R*,4*R*]-4-Amino-3-hydroxycyclohexa-1,5-diene-1-carboxylic acid (3,4-CHA) can be recovered from the fermentation broth obtained in this second embodiment of the present invention by any methods known to the skilled man. Preferably, it is recovered from the said fermentation broth by crystallization or by a separation process selected from the group consisting of reactive extraction and chromatography, optionally followed by crystallization.

The processes of the first and second embodiment of this invention can be carried out *in vivo* in a suitable host organism. When the processes according to the first and second embodiment of this invention are carried out *in vivo,* that is in living cells, prokaryotic as well as, if the biosynthetic pathway to chorismate is present therein, eukaryotic cells may be used as host cells for the process of the invention. It is to be noticed, that the process according to the second embodiment of this invention also may be carried out (enzymatically) *in vitro*.

The host organism for the *in vivo* processes according to the invention can be any host suitable for fermentation processes. Most preferably, however, the process (of the first or second embodiment of this invention) is performed in a host organism selected from the group of genera consisting of *Bacillus, Corynebacterium, Escherichia,* and *Pichia.*

The present invention further relates to expression vectors for use in a process according to any of the method claims of the present invention, where the process is carried out *in vivo.* As meant herein, expression vectors may comprise all of the required genes together on a single vector, or comprise different genes at different vectors. The vectors, or their expression cassettes (operons), or the genes pertaining thereto, may be chromosomally integrated.

The present invention, moreover, relates to host cells, especially host cells from one of the host organisms selected from the group of genera consisting of *Bacillus, Corynebacterium, Escherichia,* and *Pichia,* comprising at least one of the following activities or combinations of activities at an increased level of activity, namely of
- a PabAB bipartite protein as used according to any of the claims 3 to 8; or
- an 4-amino-4-deoxychorismate synthase (especially a PabAB bipartite protein), and a 4-amino-4-deoxyprephenate dehydrogenase and an aminotransferase; or
- an 4-amino-4-deoxychorismate synthase (especially a PabAB bipartite protein), and an enzyme capable of converting isochorismate into [5*S*,6*S*]-5,6-dihydroxycyclohexa-1,3-diene-1-carboxylic acid (2,3-CHD); or
- an 4-amino-4-deoxychorismate synthase (especially a PabAB bipartite protein), and of an enzyme capable of converting isochorismate into [5*S*,6*S*]-5,6-dihydroxycyclohexa-1,3-diene-1-carboxylic acid (2,3-CHD) and of converting 4-amino-4-deoxyisochorismate into [5*S*,6*S*]-6-amino-5-hydroxycyclohexa-1,3-diene-1-carboxylic acid (2,3-CHA); or
- a PabAB bipartite protein and a phenazine biosynthesis PhzD protein; or
- a PabAB bipartite protein and a tagged phenazine biosynthesis PhzD protein.

Of course, within the context of the present invention, the hosts used also may contain further modifications to improve the biosynthesis of the desired products. For instance, genes encoding for activities that may be competing with the chorismate activity may be deleted (e.g. deletion of the genes *tyrA*, encoding chorismate mutase/prephenate dehydrogenase, and/or *pheA*, encoding chorismate mutase/prephenate dehydratase, from the chromosome); or key enzymes of the common aromatic pathway may be overexpressed (e.g. overexpression of *aroF*, encoding DAHP synthase).

Finally, the present invention also relates to the further conversion of [3*R*,4*R*]-4-amino-3-hydroxycyclohexa-1,5-diene-1-carboxylic acid (3,4-CHA) as can be produced according to the present invention. Such further conversion advantageously can be carried out to obtain all kinds of derivatives of 3,4-CHA. A good example of such further conversion is the synthesis of oseltamivir (phosphate).

Thus far, all syntheses of oseltamivir (phosphate) require the introduction of two nitrogen functionalities in position 4 and 5 of the target molecule with the correct relative and absolute configuration. The shortest route published so far, which uses shikimate as a starting compound, requires 11 steps. This is, schematically, represented in Scheme 1 below.

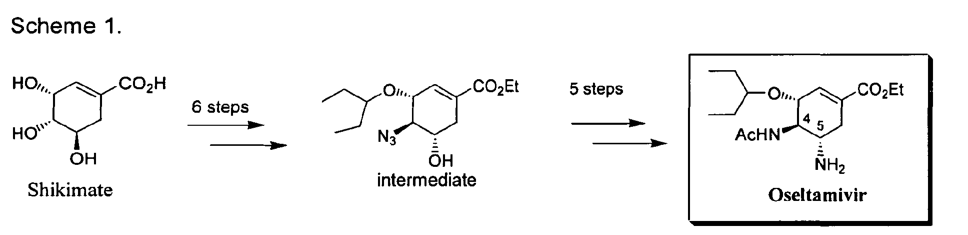

As can been seen from Scheme 2 below, ADC as well as 3,4-CHA contain the nitrogen functionality at C-4 in the right absolute and relative configuration with regard to the oxygen-substituent at C-3. Thus, the synthesis of oseltamivir (phosphate) according to the present invention, namely starting from the biosynthesis of 3,4-CHA, is highly simplified in that only the amino group at C-5 has to be introduced correctly. Introduction of the C-5 amino group can be performed e.g. via aziridination and subsequent reduction (cf. X. E. Hu, *Tetrahedron, 60,* p.2701-2743 (2004)) or via amination, either directly or via, for instance, solvomercuration and subsequent reduction according to standard techniques (e.g. as described by M. B. Gasc *et al.,* Tetrahedron 39, p.703-731 (1983)). Protection of 3,4-CHA can be performed via standard techniques. Accordingly, after such shortened series of reaction steps, which may be carried out as a sequence of individual reaction steps that can be carried out in varying orders of steps, preferably (but not necessarily) via the fully protected compound that still has to be aminated at the C-5 position (referred to in Scheme 2 as "new intermediate") as shown in Scheme 2 below. However, many different sequences of such individual reaction steps are possible starting from the 3,4-CHA prepared according to the present invention. Moreover, also 3,4-CHA prepared in another way than by biosynthesis according to the present invention can be used as starting material for the subsequent reaction steps leading to oseltamivir phosphate.

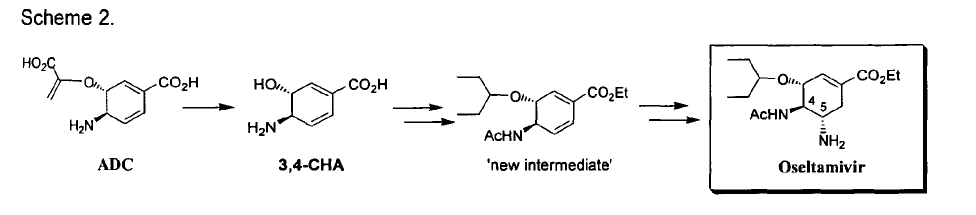

In particular, therefore, the present invention also relates to a novel method for the synthesis of oseltamivir phosphate, comprising the steps of
a) providing [3*R*,4*R*]-4-amino-3-hydroxycyclohexa-1,5-diene-1-carboxylic acid (3,4-CHA), preferably by biosynthesis of 3,4-CHA according to any of the claims relating to the synthesis of 3,4-CHA; and,
   in any order of steps b) - e)
b) esterification of the carboxylic function to its ethyl ester;
c) etherification of the hydroxylic function into its 3-pentanol ether;
d) acetylation of the amino group at C-3;
e) introduction of an amino function in *trans*-position with respect to the N-containing group at C-4;
   followed by
f) conversion of the product prepared from 3,4-CHA as a result of the combined reaction steps b), c), d), and e) into oseltamivir phosphate.

A reaction that, for instance, suitably can be applied in the step b), esterification, has been described by Federspiel *et al*,, Organic Process & Research Development 1999, p.266-274, especially the conversion of intermediate 23 into 24 in the first step of the shikimic acid route at page 273. Etherification in step c) may be done by all methods known to the skilled man. Acetylatation in step d), for instance, can be performed using a method described by Karpf *et al.,* J. Org. Chem. 66, p.2044-2051 (2001), in particular in the conversion of intermediate 17 into 18 at page 2049. Introduction of an amino function as in step e), for instance, may be carried out by aziridination or amination (see the article of X. E. Hu, respectively that of M. B. Gasc *et al*., mentioned above). The final conversion into oseltamivir phosphate, for instance, can be done as described by Carr *et al.,* J. Org. Chem., 62, p.8640-8653 (1997), in particular the conversion of intermediate 12 into 13 at page 8648.

The attached formulae sheet, preceding the experimental part, shows the structures of various of the compounds mentioned in this application, namely:
Chorismate
Isochorismate
3,4-CHA (trans position of amino and hydroxy group)
2,3-CHA (trans position of amino and hydroxy group)
3,4-CHD (trans position of hydroxy groups)
2,3-CHD (trans position of hydroxy groups)
ADC
ADIC
ADP
p-aminophenylalanine.

The invention hereinafter will be explained by means of a number of examples examples, which by no means are intended to restrict the scope of the present invention.

Sequence listings of nucleotides referred to in the experimental part, namely [SEQ ID: No.1] to [SEQ ID: No.10], are shown at the end of the experimental part. They also will be submitted in electronic format (Patentln).

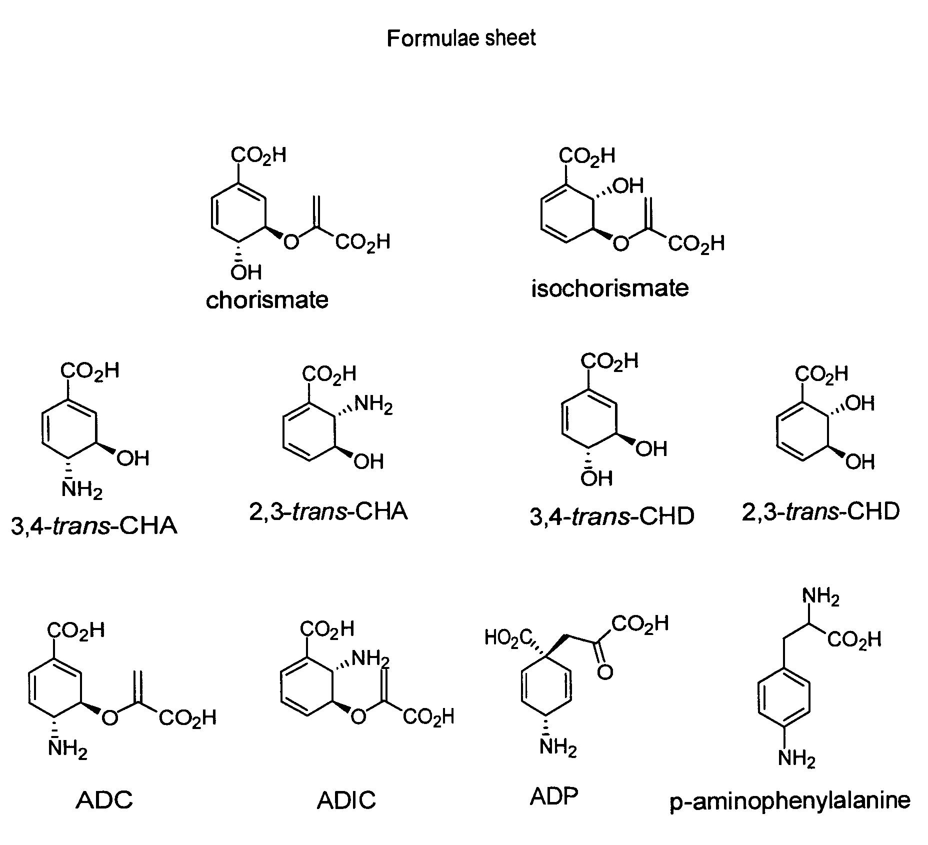

### Experimental part

### Experimental Part I (Examples 1-8): Cloning of single genes and construction of ADC and 3,4-CHA biosynthesis operons

### General procedures for Examples 1-8

Standard molecular cloning techniques such as plasmid DNA isolation, gel electrophoresis, enzymatic restriction modification of nucleic acids, *E. coli* transformation etc. were performed as described by Sambrook *et al*., 1989, "Molecular Cloning: a laboratory manual", Cold spring Harbor Laboratories, Cold Spring Harbor, NY. Synthetic oligodeoxynucleotides were obtained from MWG-Biotech AG (Ebersberg, Germany). DNA sequence analyses were performed by GATC Biotech AG (Konstanz, Germany) and AGOWA GmbH (Berlin, Germany).

### General considerations for Examples 1-8

As expression vector for the ADC and 3,4-CHA biosynthesis genes, plasmid pJF119EH was chosen; Fürste, et al. (1986, Gene, 48:119-131). The expression system uses the IPTG inducible tac promoter and carries the lac repressor (*lac*I^{q} gene), which keeps the expression of the cloned foreign gene in the absence of the inducer extremely low. Additionally, the *phzD* gene of *P. aeruginosa* was cloned in expression vector pET16b (Calbiochem-Novabiochem GmbH, Schwalbach/Ts., Germany) producing a PhzD protein that contains an N-terminal His•Tag® sequence and the protease Factor Xa recognition site.

### Example 1: Construction of plasmid pC26

Genomic DNA from *Pseudomonas aeruginosa* ATCC 17933 was obtained from the American Type Culture Collection (ATCC), Manassas, VA, USA. A 706 bp fragment comprising the open reading frame (ORF) for PA1902, encoding the phenazine biosynthesis protein PhzD was amplified by PCR from the chromosomal DNA from *Pseudomonas aeruginosa* ATCC 17933 (nucleotides 3857-4480 of accession number AE004616; amplified region nucleotides 3840-4480) using the following primers: 5' - GAGCGCAAAGCTTCCCCTGGAGACCGTGGCATGAGC - 3' [SEQ ID: No. 1] (with *Hin*dIII recognition site underlined) and 5' - GTGGGCAAAGCTTTCATTCCAGCACCTCGTCGGTGG - 3'   [SEQ ID: No. 2] (with *Hin*dlll recognition site underlined).

A list of all nucleotide sequences used in the context of the present application is presented in the SEQUENCE LISTING annexed hereto.

Correct size of the amplified fragment was confirmed by agarose gel electrophoresis and the fragment was purified from the gel. The fragment was digested with the enzyme *Hin*dIII to generate sticky ends. The plasmid pJF119EH was digested with *Hin*dIII and dephosphorylated. The two fragments were subsequently ligated and used for the transformation of chemically competent cells of *E. coli* DH5α (Invitrogen GmbH, Karlsruhe, Germany). The transformants were selected on LB agar plates containing 100 mg·l⁻¹ ampicillin. A plasmid showing the correct insert sequence (as confirmed by sequencing) was called pC26.

It is to be noticed that the *phzD* gene occurs twice in the genome of *Pseudomonas aeruginosa.* An exact copy can be found under accession number AE004838 nucleotides 2623-3246 (PA4213).

### Example 2: Construction of plasmid pC49

The *P. aeruginosa phzD* gene encoding the phenazine biosynthesis protein PhzD was subcloned in pET16b using PCR. The PhzD ORF was amplified using 5' - CCCCTGGAGACCGTCATATGAGCGGCATTCCCG - 3'     [SEQ ID: No. 3] (with *Nde*l recognition site underlined) and 5' - CCGGTTCTGCGTTCTGATTTGGATCCTATCAGGC - 3'     [SEQ ID: No. 4] (with *Bam*HI recognition site underlined)
as primers and pC26 (see example 1) plasmid DNA as template.

Correct size (712 bp) of the amplified fragment was confirmed by agarose gel electrophoresis. The fragment and the plasmid pET16b were digested with *Nde*l and *Bam*HI. The two fragments were ligated and transformed in *E. coli* DH5α. Transformants were selected on LB agar plates containing 100 mg·l⁻¹ ampicillin. A plasmid showing the correct insert sequence (as confirmed by sequencing) was called pC49.

### Example 3: Construction of plasmid pC53

Genomic DNA was prepared from *Corynebacterium glutamicum* ATCC 13032, which was obtained from the American Type Culture Collection (ATTC), Manassas, VA, USA. A 1907 bp fragment comprising the ORF (Cgl0997) for the aminodeoxychorismate synthase was amplified by PCR from *Corynebacterium glutamicum* ATCC 13032 chromosomal DNA (nucleotides 1052000-1053883 of accession number NC 003450; amplified region nucleotides 1052000-1053888) using the following primers: 5' - TTTTAGATCTGTGGTTTTGTCAGAGGATGTC - 3'     [SEQ ID: No. 5] (with *Bgl*II recognition site underlined) and 5' - TTGGATCCGTACGTCATGGaAATTCAAC - 3'          [SEQ ID: No. 6] (with *Bam*HI recognition site underlined and changed nucleotide, indicated by lower case letter, in order to destroy the internal *Eco*RI recognition site).

Correct size of the amplified fragment was confirmed by agarose gel electrophoresis and the fragment was purified from the gel. The fragment was digested with the enzyme *Bgl*II and *Bam*HI to generate sticky ends. The plasmid pJF119EH was digested with *Bam*HI and dephosphorylated. The two fragments were subsequently ligated and used for the transformation of chemically competent cells of *E. coli* DH5α. The transformants were selected on LB agar plates containing 100 mg·l⁻¹ ampicillin. A plasmid showing the correct insert sequence (as confirmed by sequencing) was called pC53.

### Example 4: Construction of plasmid pC56

In this example, the cloned *pabAB* and *phzD* from examples 1 and 3 were combined in the expression vector pJF119EH. The *pabAB* gene in pC53 was excised from the expression vector by digestion with *Eco*RI and *Bam*HI, and this DNA fragment containing the *pabAB* gene was purified by gel electrophoresis. Plasmid pC26 was digested with *Eco*RI and *Bam*HI, ligated together with the PabAB *Eco*RI /*Bam*HI fragment and transformed in *E. coli* DH5α. Transformants were selected on LB agar plates containing 100 mg·l⁻¹ ampicillin. A plasmid comprising the two genes in the correct order in pJF119EH (as confirmed by restriction mapping) was designated pC56.

### Example 5: Construction of plasmid pC78

In this example, the cloned *pabAB* and *phzD* from examples 2 and 3 were combined in the expression vector pJF119EH. The *phzD* gene in pC49 was excised from the expression vector by digestion with *Bgl*II and *Bam*HI, and this DNA fragment containing the *phzD* gene was purified by gel electrophoresis. Plasmid pC53 was digested with *Bam*HI, dephosphorylated, ligated together with the PhzD *Bgl*II / *Bam*Hl fragment and transformed in *E. coli* DH5α. Transformants were selected on LB agar plates containing 100 mg·l⁻¹ ampicillin. A plasmid comprising the two genes in the correct order in pJF119EH (as confirmed by restriction mapping) was designated pC78.

### Example 6: Construction of plasmid pF34

Genomic DNA was prepared from *E*. *coli* W3110 strain LJ110 (T. Zeppenfeld *et al.,* J.Bacteriol. 182 (2000), pages 4443-4452). A 1213 bp fragment comprising the gene *aroF* for the DAHP synthase (tyr) was amplified by PCR from *E. coli* LJ110 chromosomal DNA (nucleotides 5872-6942 of accession number AE000346; amplified region nucleotides 5786-6965) using the following primers: 5' - CTATCGAATTCGAGCATAAACAGGATCGCC - 3'     [SEQ ID: No. 7] (with *Eco*RI recognition site underlined) and 5' - CACTTCAGCAACCAGTTCCCGGGGCTTCGC - 3'     [SEQ ID: No. 8] (with *Sma*l recognition site underlined).

Correct size of the amplified fragment was confirmed by agarose gel electrophoresis. The fragment and the plasmid pJF119EH were digested with *Eco*RI and *Sma*I. The two fragments were ligated and transformed in *E. coli* DH5α. Transformants were selected on LB agar plates containing 100 mg·l⁻¹ ampicillin. A plasmid showing the correct insert sequence (as confirmed by sequencing) was called pF34.

### Example 7: Construction of plasmids pC99, pC100, pC101

In this example, the cloned *pabAB* and *phzD* from examples 3, 4, and 5 were combined with *aroF* in the expression vector pJF119EH. The plasmid pF34 was digested with the enzymes *Sma*I and *Sca*I and the 5595 bp fragment was purified from the gel.

### 7.1 Construction of plasmid pC99

The plasmid pC53 was digested with *Eco*RI and the sticky ends were filled in using Klenow enzyme. Subsequently, the *pabAB* gene was excised by digestion with *Sca*I and the 2774 bp DNA fragment was purified by gel electrophoresis. The 5595 bp DNA fragment from pF34 and the 2774 bp DNA fragment were ligated and transformed in *E. coli* DH5α. Transformants were selected on LB agar plates containing 100 mg·l⁻¹ ampicillin. A plasmid comprising the aroF and *pabAB* genes (as confirmed by restriction mapping) was designated pC99.

### 7.2 Construction of plasmid pC100

The plasmid pC56 was digested with *Eco*RI and the sticky ends were filled in using Klenow enzyme. Subsequently, the *pabAB-phzD* DNA fragment was excised by digestion with *Sca*I and the 3421 bp DNA fragment was purified by gel electrophoresis. The 5595 bp DNA fragment from pF34 and the 3421 bp DNA fragment were ligated and transformed in *E. coli* DH5α. Transformants were selected on LB agar plates containing 100 mg·l⁻¹ ampicillin. A plasmid comprising the *aroF*, *pabAB*, and *phzD* genes (as confirmed by restriction mapping) was designated pC100.

### 7.3 Construction of plasmid pC101

The plasmid pC78 was digested with *Eco*RI and the sticky ends were filled in using Klenow enzyme. Subsequently, the *pabAB-phzD* DNA fragment was excised by digestion with *Sca*I and the 3613 bp DNA fragment was purified by gel electrophoresis. The 5595 bp DNA fragment from pF34 and the 3613 bp DNA fragment were ligated and transformed in *E*. *coli* DH5α. Transformants were selected on LB agar plates containing 100 mg·l⁻¹ ampicillin. A plasmid comprising the *aroF, pabAB*, and *phzD* genes (as confirmed by restriction mapping) was designated pC101.

### Example 8: Construction of host strain F4

The *pheA tyrA aroF* gene locus of *E*. *coli* W3110 strain LJ110 (T. Zeppenfeld *et al*., J.Bacteriol. 182 (2000), pages 4443-4452) was inactivated according to K. A. Datsenko and B. L. Wanner, PNAS 97 (2000), pages 6640-6645. The inactivation cassette was amplified by PCR from the plasmid pKD4 using the following primers:

5' - AACTTCGTCGAAGAAGTTGAAGAAGAGTAGTCCTTTATATTGAGTGTATCGT-
GTAGGCTGGAGCTGCTTC- 3' [SEQ ID: No. 9]

5' - AGTGTAAATTTATCTATACAGAGGTAAGGGTTGAAAGCGCGACTAAATTGCA-
TATGAATATCCTCCTTAG- 3' [SEQ ID: No. 10]

A strain carrying the deletion of the *pheA tyrA aroF* gene locus was designated F4.

### Experimental Part II (Examples 9-12): Production of ADC + ADP, and 3,4-CHA

### Example 9: Fermentative production of mixture of ADC and ADP in fed-batch bioreactor

The ADC production of *E. coli* F4/pC99 from glucose was investigated in mineral medium. The precultivation medium consisted of MgSO₄·7H₂O (0.3 g·l⁻¹), CaCl₂·2H₂O (0.015 g·l⁻¹), KH₂PO₄ (3.0 g·l⁻¹), K₂HPO₄ (12.0 g·l⁻¹), NaCl (0.1 g·l⁻¹), (NH₄)₂SO₄ (5.0 g·l⁻¹), FeSO₄·7H₂O (0.075 g·l⁻¹), Na-citrate·3H₂O (1.0 g·l⁻¹), thiamine·HCl (0.0125 g·l⁻¹), L-tyrosine (0.05 g·l⁻¹), and L-phenylalanine (0.05 g·l⁻¹). Additional minerals were added in the form of a trace element solution (1.0 ml·l⁻¹), which trace element solution was composed of Al₂(SO₄)₃·18H₂O (2.0 g·l⁻¹), CoCl₂·6H₂O (0.75 g·l⁻¹), CuSO₄·5H₂O (2.5 g·l⁻¹), H₃BO₃ (0.5 g·l⁻¹), MnCl₂·4H₂O (20.0 g·l⁻¹) Na₂MoO₄·2H₂O (3.0 g·l⁻¹), NiSO₄·6H₂O (20 g·l⁻¹), ZnSO₄·7H₂O (15.0 g·l⁻¹). A glucose stock solution (500 g·l⁻¹) was autoclaved separately and added to the sterilized medium to a final concentration of 15 g·l⁻¹.

The stock culture of *E. coli* F4/pC99 was stored at -80°C in Luria-Bertani (LB) medium containing 50% glycerol. 1 ml feedstock was used to inoculate 200 ml of precultivation medium containing ampicillin (100 mg·l⁻¹) split in two 1 I shaking flasks and incubated at 37°C and 180 rpm for 16 hours.

The fermentation medium was the same as for precultivation except for the following changes: MgSO₄·7H₂O (0.9 g·l⁻¹), K₂HPO₄ was omitted, FeSO₄·7H₂O (0.1125 g·l⁻¹), Na-citrate·3H₂O (1.5 g·l⁻¹), thiamine·HCl (0.075 g·l⁻¹), L-tyrosine (0.15 g·l⁻¹), and L-phenylalanine (0.3 g·l⁻¹). Additional minerals were added in the form of a trace element solution (1.5 ml·l⁻¹).

Fed-batch cultivation was performed in 5 l Labfors bioreactor (Infors, Einsbach, Switzerland) with 10% inoculation for 27.25 h; 2 I initial volume, cultivation temperature 37°C, pH 6.8. pH was controlled by 12.5 % ammonia and 2.5N KOH titration. After 8.25 h a feed for L-tyrosine and L-phenylalanine (5 g·l⁻¹ L-tyrosine, 6 g·l⁻¹ L-phenylalanine dissolved in 1N KOH; feed rate 7 g·h⁻¹) was started. After 9.25 h a feed for glucose (500 g·l⁻¹; feed rate 12.5 g·h⁻¹) was started. After 7.5 h at an OD₆₂₀ₙₘ of ~ 4.0 ADC production was induced by addition of 0.1 mM IPTG.

A sample of the culture supernatant was lyophilized and redissolved in D₂O. 600 MHz ¹H-NMR at 303 K showed the expected resonance spectrum. The assignment of all the resonances was done by using several 2D NMR techniques (¹H-¹H COSY, ¹H-¹H TOCSY, ¹H-¹³C COSY and ¹H-¹³C long range HMBC). All the spectra confirmed the presence of ADC and ADP. The amount of ADC present was determined to be 7 g·l⁻¹. (Besides ADC and ADP, 16 g·l⁻¹ of its precursor chorismate was present as determined by HPLC analysis.)

In separate batches mixtures of ADC and ADP were isolated from fermentation supernatant by ion exchange chromatography (Dowex 50Wx8, H⁺-form,.elution with 0.5 M ammonia solution) in varying ratios of ADC and ADP, often with an excess of ADP. Chorismate was removed quantitatively. The final product contained roughly 1:1 of ADC and ADP.

### Example 10: In vitro production of 3,4-CHA

Single colonies of *E. coli* strain BL21(DE3)pLysS (Calbiochem-Novabiochem GmbH, Schwalbach/Ts., Germany) harboring the plasmid pC49 (see Example 2) were cultivated in 200 ml LB medium containing ampicillin (100 mg·l⁻¹) at 37°C. At OD₆₀₀ₙₘ of 0.6, the cells were induced by the addition of IPTG (1 mM). After 4 h the cells were harvested, resuspended in buffer (20 mM N-(2-hydroxyethyl)-piperazine-N'-(2-ethanesulfonic acid (HEPES), 300 mM NaCl, 1 mM dithiothreitol (DTT), 20 mM imidzole) and frozen at -70°C for later use.

The frozen cells were thawed at 30°C in a water bath and tehn incubated for 1 h on ice for complete lysis. Cell debris was removed by centrifugation and the resulting cell free extract was applied onto a 25 ml Ni-NTA Superflow column (Qiagen, Hilden, Germany). After a washing step (20 mM HEPES, 300 mM NaCl, 1 mM DTT, 20 mM imidazole) the His•PhzD protein was obtained in at least 95% purity by elution with buffer (20 mM HEPES, 300 mM NaCl, 1 mM DTT) containing increasing concentrations of imidazole (max. 250 mM). The partially purified extract was used in further investigation.

The 3,4-CHA production assay of 3 ml contained 0.2 M K-phosphate buffer (pH 7.6), 85 mg ADC, and 62.5 µg partially purified His•PhzD. The assay was started by addition of His•PhzD and stopped after 2 h at 37°C. The protein was removed by centrifugation through a Centricon 10 column (Milipore, Eschborn, Germany). The reaction mixture was separated by ion exchange chromatography. A sample was dissolved in D₂O and analyzed by 300 MHz ¹H-NMR at 293K confirming the presence of 3,4-CHA.

### Example 11: Fermentative production of 3,4-CHA in shake flask

The 3,4-CHA production of *E. coli* strains F4/pC100 and F4/pC101, respectively, from glucose was investigated in mineral medium. This mineral medium was the same as the precultivation medium as described in example 9, except for glucose (10 g·l⁻¹).

The stock cultures of *E. coli* F4/pC100 and F4/pC101 were stored at -80°C in Luria-Bertani (LB) medium containing 50% glycerol. 1.8 ml feedstock was used to inoculate 50 ml of minimal medium containing ampicillin (100 mg·l⁻¹) in a 500 ml shaking flask and incubated at 37°C and 180 rpm for 16 hours. 125 µl of this culture was subsequently used to inoculate 50 ml of the same medium in a 500 ml shaking flask and incubated at 37°C and 180 rpm for 24 h. After 3.25 h at an OD₆₂₀ₙₘ of ~1.5. the cells were induced by adding 0.1 mM IPTG.

A sample of the culture supernatant was lyophilized and redissolved in D₂O. 600 MHz ¹H-NMR at 303 K showed the expected resonance spectrum. The assignment of all the resonances was done by using several 2D NMR techniques (¹H-¹H COSY, ¹H-¹H TOCSY, ¹H-¹³C COSY and ¹H-¹³C long range HMBC). All the spectra confirmed the presence of 3,4-CHA. The amount present was determined to be 30 mg·l⁻¹ and 150 mg·l⁻¹ for the strains F4/pC100 and F4/pC101, respectively. (Besides 3,4-CHA, 102 mg·l⁻¹ and 520 mg·l⁻¹, respectively, of 3,4-CHD were present, but no ADC, ADP, and p-aminobenzoic acid as determined by HPLC and NMR analysis.)

### Example 12: Fermentative production of 3,4-CHA in fed-batch bioreactor

The precultivation of F4/pC101 was performed as described in example 9. The fermentation medium was the same as in example 9.

Fed-batch cultivation was performed in 5 l Labfors bioreactor (Infors, Einsbach, Switzerland) with 10% inoculation for 31.25 h; 2 I initial volume, cultivation temperature 37°C, pH 6.8. pH was controlled by 12.5 % ammonia and 2.5N KOH titration. After 7.5 h a feed for L-tyrosine and L-phenylalanine (5 g·l⁻¹ L-tyrosine, 6 g·l⁻¹ L-phenylalanine dissolved in 1 N KOH; feed rate 7 g·h⁻¹) was started. After 6.5 h a feed for glucose (500 g·l⁻¹; feed rate 12.5 g·h⁻¹) was started. After 7.0 h at an OD₆₂₀ₙₘ of ∼7.5 3,4-CHA production was induced by addition of 0.1 mM IPTG. The supernatant was analyzed for 3,4-CHA by ¹H-NMR and the amount present was determined to be 1.7 g·l⁻¹. Besides said amount of 3,4-CHA, 5.4 g·l⁻¹ of 3,4-CHD was found to be present, as well as very minor amounts, at about their detection limits, of ADC (< 37 mg·l⁻¹) and of p-aminobenzoic acid (< 27 mg·l⁻¹) were present as determined by HPLC analysis.

3,4-CHA was isolated, in a yield of about 90% by weight, from fermentation supernatant by ion exchange chromatography.

All sequences of nucleotides referred to in the experimental part are listed hereinafter, and will be submitted in electronic format (Patentln).

Sequence listings of oligonucleotides

[SEQ ID: No.1]
GAGCGCAAAG CTTCCCCTGG AGACCGTGGC ATGAGC 36

[SEQ ID: No.2]
GTGGGCAAAG CTTTCATTCC AGCACCTCGT CGGTGG 36

[SEQ ID: No.3]
CCCCTGGAGA CCGTCATATG AGCGGCATTC CCG 33

[SEQ ID: No.4]
CCGGTTCTGC GTTCTGATTT GGATCCTATC AGGC 34

[SEQ ID: No.5]
TTTTAGATCT GTGGTTTTGT CAGAGGATGT C 31

[SEQ ID: No.6]
TTGGATCCGT ACGTCATGGA AATTCAAC 28

[SEQ ID: No.7]
CTATCGAATT CGAGCATAAA CAGGATCGCC 30

[SEQ ID: No.8]
CACTTCAGCA ACCAGTTCCC GGGGCTTCGC 30

[SEQ ID: No.9]
AACTTCGTCG AAGAAGTTGA AGAAGAGTAG TCCTTTATAT TGAGTGTATC GTGTAGGCTG
GAGCTGCTTC 70

[SEQ ID: No.10]
AGTGTAAATT TATCTATACA GAGGTAAGGG TTGAAAGCGC GACTAAATTG CATATGAATA
TCCTCCTTAG 70

<210> 4
<211> 34
<212> DNA
<213> Artificial sequence

<220>
<223> primer

<400> 4
ccggttctgc gttctgattt ggatcctatc aggc
34


<210> 5
<211> 31
<212> DNA
<213> Artificial sequence

<220>
<223> primer

<400> 5
ttttagatct gtggttttgt cagaggatgt c
31


<210> 6
<211> 28
<212> DNA
<213> Artificial sequence

<220>
<223> primer

<400> 6
ttggatccgt acgtcatgga aattcaac
28


<210> 7
<211> 30
<212> DNA
<213> Artificial sequence

<220>
<223> primer

<400> 7
ctatcgaatt cgagcataaa caggatcgcc
30


<210> 8
<211> 30
<212> DNA

<213>  Artificial sequence

<220>
<223>  primer

<400>  8
cacttcagca accagttccc ggggcttcgc
    30


<210>  9
<211>  70
<212>  DNA
<213>  Artificial sequence

<220>
<223>  primer

<400>  9
aacttcgtcg aagaagttga agaagagtag tcctttatat tgagtgtatc gtgtaggctg
    60

gagctgcttc
    70


<210>  10
<211>  70
<212>  DNA
<213>  Artificial sequence

<220>
<223>  primer

<400>  10
agtgtaaatt tatctataca gaggtaaggg ttgaaagcgc gactaaattg catatgaata
    60

tcctccttag
    70

SEQUENCE LISTING

<110> DSM Biotech GmbH
DSM IP Assets B.V.
Forschungszentrum Jülich GmbH

<120> Biosynthetic production of 4-amino-4-deoxychorismate (ADC)
and [3R,4R]-4-amino-3-hydroxycyclohexa-1,5-diene-1-carboxylic acid
(3,4-CHA)

<130> 21583EP/P0

<160> 10

<170> PatentIn version 3.1

<210> 1
<211> 36
<212> DNA
<213> Artificial sequence

<220>
<223> primer

<400> 1
gagcgcaaag cttcccctgg agaccgtggc atgagc
36

<210> 2
<211> 36
<212> DNA
<213> Artificial sequence

<220>
<223> primer

<400> 2
gtgggcaaag ctttcattcc agcacctcgt cggtgg
36

<210> 3
<211> 33
<212> DNA
<213> Artificial sequence

<220>
<223> primer

<400> 3
cccctggaga ccgtcatatg agcggcattc ccg
33

## Claims

1. Process for the biosynthetic production of 4-amino-4-deoxychorismate (ADC) catalyzed at least by an enzyme belonging to the class of aminodeoxychorismate synthases, **characterized in that** the biosynthetic production is performed fermentatively *in vivo* with a 4-amino-4-deoxychorismate synthase at an increased level of activity , while obtaining a fermentation broth comprising 4-amino-4-deoxychorismate (ADC) and 4-amino-4-deoxyprephenate (ADP),
and that these compounds, either together or individually, are recovered from the fermentation broth.

2. Process according to claim 1, **characterized in that**
the 4-amino-4-deoxychorismate synthase is a PabAB bipartite protein.

3. Process according to claim 2, **characterized in that**
the bipartite protein originates from a species from the group of *Actinomycetes,* or from plants containing such bipartite enzyme, or is constructed by fusion of genes respectively encoding for PabA and PabB.

4. Process according to claim 2 or 3, **characterized in that**
the PabAB bipartite protein originates, as such or as a fusion protein, from a species from the group of genera consisting of *Escherichia, Corynebacterium, Saccharomyces* and *Streptomyces.*

5. Process according to claim 4, **characterized in that**
the PabAB bipartite protein originates, as such or as a fusion protein, from one of the species from the group of species consisting of *Escherichia coli, Corynebacterium glutamicum, Corynebacterium diphtheriae gravis* NCTC13129, *Corynebacterium efficiens, Saccharomyces cerevisiae, Streptomyces griseus, Streptomyces venezuelae, Streptomyces sp*.FR-008, *Streptomyces pristinaespiralis, Streptomyces thioluteus,* and *Streptomyces avermitilis*.

6. Process according to claim 5, **characterized in that**
the PabAB bipartite protein originates from *Corynebacterium glutamicum* ATCC 13032.

7. Process according to any of claims 1 to 8, **characterized in that**
the 4-amino-4-deoxychorismate (ADC) and 4-amino-4-deoxyprephenate (ADP) formed are recovered from the said fermentation broth, together or individually, by a separation process selected from the group consisting of reactive extraction and chromatography, optionally followed by crystallization.

8. Process according to any of claims 1 to 6, **characterized in that**
the 4-amino-4-deoxyprephenate (ADP) in the mixture of 4-amino-4-deoxychorismate (ADC) and 4-amino-4-deoxyprephenate (ADP) is converted into p-aminophenylalanine by means of concerted action, and at an increased level of activity, of a 4-amino-4-deoxyprephenate dehydrogenase and an aminotransferase.

9. Process for the biosynthetic production of [3*R*,4*R*]-4-amino-3-hydroxycyclohexa-1,5-diene-1-carboxylic acid (3,4-CHA), **characterized in that**
the biosynthetic production is performed by concerted action, and at an increased level of activity, of a 4-amino-4-deoxychorismate synthase and of an enzyme capable of converting isochorismate into [5*S*,6*S*]-5,6-dihydroxycyclohexa-1,3-diene-1-carboxylic acid (2,3-CHD), and that the [3*R*,4*R*]-4-amino-3-hydroxycyclohexa-1,5-diene-1-carboxylic acid (3,4-CHA) is recovered from the fermentation broth so obtained.

10. Process according to claim 9, **characterized in that**
the biosynthetic production is performed fermentatively *in vivo.*

11. Process according to claim 9 or 10, **characterized in that** the 4-amino-4-deoxychorismate synthase is a PabAB bipartite protein.

12. Process according to claim 11, **characterized in that**
the PabAB bipartite protein is a protein as described, in or according to any of claims 3 to 6, and **in that**
the enzyme capable of converting isochorismate into [5*S*,6*S*]-5,6-dihydroxycyclohexa-1,3-diene-1-carboxylic acid (2,3-CHD) is also capable of converting 4-amino-4-deoxyisochorismate into [5*S*,6*S*]-6-amino-5-hydroxycyclohexa-1,3-diene-1-carboxylic acid (2,3-CHA).

13. Process according to claim 11 or 12, **characterized in that**
the enzyme capable of converting isochorismate into [5*S*,6*S*]-5,6-dihydroxycyclohexa-1,3-diene-1-carboxylic acid (2,3-CHD) is a phenazine biosynthesis PhzD protein.

14. Process according to claim 13, **characterized in that**
the phenazine biosynthesis PhzD protein originates from a species from the group of genera consisting of *Pseudomonas, Pantoea, Streptomyces,* and *Enwinia*.

15. Process according to any of claims 13 or 14, **characterized in that**
the phenazine biosynthesis PhzD protein originates from a species selected from the group consisting of *Pseudomonas aeruginosa, Pseudomonas aureofaciens, Pseudomonas fluorescens, Pseudomonas chlororaphis,* and *Pantoea agglomerans* species.

16. Process according to any of claims 13 to 15, **characterized in that**
the phenazine biosynthesis PhzD protein originates from *Pseudomonas aeruginosa* ATCC 17933.

17. Process according to any of claims 11 to 16, **characterized in that**
the PabAB bipartite protein originates from a species selected from the group of species consisting of *Escherichia coli, Corynebacterium glutamicum, Corynebacterium diphtheriae gravis* NCTC13129, *Corynebacterium efficiens, Saccharomyces cerevisiae, Streptomyces griseus, Streptomyces venezuelae, Streptomyces sp.* FR-008, *Streptomyces pristinaespiralis, Streptomyces thioluteus,* and *Streptomyces avermitilis*.

18. Process according to claim 17, **characterized in that**
the PabAB bipartite protein originates from *Corynebacterium glutamicum* ATCC 13032.

19. Process according to any of claims 9 to 18, **characterized in that**
the [3*R*,4*R*]-4-amino-3-hydroxycyclohexa-1,5-diene-1-carboxylic acid (3,4-CHA) formed is recovered from the said reaction mixture by crystallization or by a separation process selected from the group consisting of reactive extraction and chromatography, optionally followed by crystallization.

20. Process according to any of claims 1 to 21, **characterized in that**
the process is performed in a host organism selected from the group of genera consisting of *Bacillus, Corynebacterium, Escherichia,* and *Pichia.*

21. Expression vector for use in a process according to any of claims 1 to 20.

22. Host cells, especially host cells from one of the host organisms selected from the group of genera consisting of *Bacillus, Corynebacterium, Escherichia,* and *Pichia,* comprising at least one of the following activities or combinations of activities at an increased level of activity, namely of
• a PabAB bipartite protein as used according to any of the claims 3 to 6; or
• an 4-amino-4-deoxychorismate synthase (especially a PabAB bipartite protein), and a 4-amino-4-deoxyprephenate dehydrogenase and an aminotransferase; or
• an 4-amino-4-deoxychorismate synthase (especially a PabAB bipartite protein), and an enzyme capable of converting isochorismate into [5*S*,6*S*]-5,6-dihydroxycyclohexa-1,3-diene-1-carboxylic acid (2,3-CHD); or
• an 4-amino-4-deoxychorismate synthase (especially a PabAB bipartite protein), and of an enzyme capable of converting isochorismate into [5*S*,6*S*]-5,6-dihydroxycyclohexa-1,3-diene-1-carboxylic acid (2,3-CHD) and of converting 4-amino-4-deoxyisochorismate into [5*S*,6*S*]-6-amino-5-hydroxycyclohexa-1,3-diene-1-carboxylic acid (2,3-CHA), or
• a PabAB bipartite protein and a phenazine biosynthesis PhzD protein; or
• a PabAB bipartite protein and a tagged phenazine biosynthesis PhzD protein.

23. Method for the synthesis of oseltamivir phosphate, comprising the steps of
a) providing [3*R*,4*R*]-4-amino-3-hydroxycyclohexa-1,5-diene-1-carboxylic acid (3,4-CHA), preferably by biosynthesis of 3,4-CHA according to any of the claims relating to the synthesis of 3,4-CHA; and,
in any order of steps b) - e)
b) esterification of the carboxylic function to its ethyl ester;
c) etherification of the hydroxylic function into its 3-pentanol ether;
d) acetylation of the amino group at C-3;
e) introduction of an amino function in *trans*-position with respect to the N-containing group at C-4;
followed by
f) conversion of the product prepared from 3,4-CHA as a result of the combined reaction steps b), c), d), and e) into oseltamivir phosphate.
